# EUROPEAN PATENT APPLICATION

(11) **EP 4 116 424 A1**
(43) Date of publication of application: **11.01.2023**
(21) Application number: 21765318.7
(22) Date of filing: 26.02.2021
(51) Int. Cl.: C12N 15/54, A23L 5/00, A61K 38/45, A61P 43/00, C12N 1/15, C12N 1/19, C12N 1/21, C12N 5/10, C12N 9/10

(54) **MODIFIED TRANSGLUTAMINASE**

(30) Priority: 03.03.2020 JP 2020035920
(71) Applicant: Amano Enzyme Inc., Nagoya-shi Aichi 460-8630 (JP)
(72) Inventor: YOSHIDA, Kazunori, Kakamigahara-shi, Gifu 509-0109 (JP)
(74) Representative: Müller-Boré & Partner Patentanwälte PartG mbB
(86) International application number: PCT/JP2021/007453
(87) International publication number: WO 2021/177181

(57) **Abstract**

The present invention addresses the issues of finding a novel mutation effective for the improvement of transglutaminase and providing a highly useful modified transglutaminase. Disclosed is a highly useful modified transglutaminase having an amino acid substitution that results in an increase of high temperature reactivity or a lowering in pH stability in a weakly acidic region.

## Description

### TECHNICAL FIELD

The present invention relates to a transglutaminase. Specifically, the present invention relates to a modified transglutaminase having changed or improved properties, uses thereof, and the like.

### BACKGROUND ART

Transglutaminase is an enzyme that catalyzes an acyl transfer reaction of a γ-carboxylamide group of a glutamine residue in a peptide chain, and when an ε-amino group of a lysine residue in a protein acts as an acyl receptor, an ε-(γ-Gln)-Lys crosslinking bond is formed in a protein molecule or between protein molecules. Therefore, since it is possible to modify a protein or peptide by utilizing the action of transglutaminase, transglutaminase derived from the genus Streptomyces (see, for example, Patent Document 1) is used for binding meat, and for producing sausages, bean curds, breads, and noodles. In addition, the use of transglutaminase has been studied not only in the food field but also in the fiber field, the medical field, the cosmetic field, and the like. With such expanded use, attempts have been made to improve the properties (heat resistance, specific activity, substrate specificity, stability, and the like) of transglutaminase (see, for example, Patent Documents 2 to 4 and Non-Patent Documents 1 to 4).

### PRIOR ART DOCUMENTS

### PATENT DOCUMENTS

Patent Document 1: Japanese Patent Laid-open Publication No. H4-108381
Patent Document 2: Japanese Patent Laid-open Publication No. 2002-253272
Patent Document 3: Japanese Patent Laid-open Publication No. 2008-194004
Patent Document 4: WO2019/107288A

### NON-PATENT DOCUMENTS

Non-Patent Document 1: Marx CK et al., J. Biotechnol. 2008 Sep 10;136(3-4): 156-62.
Non-Patent Document 2: Tagami U et al., Protein Eng. Des. Sel. 2009 Dec; 22(12): 747-752.
Non-Patent Document 3: Yokoyama K et al., Appl. Microbiol. Biotechnol. (2010) 87: 2087-2096.
Non-Patent Document 4: Buettner K et al., Amino Acids. 2012 Feb;42(2-3): 987-96.

### SUMMARY OF THE INVENTION

### PROBLEMS TO BE SOLVED BY THE INVENTION

As described above, attempts have been made so far to improve transglutaminase for the purpose of, for example, improving heat resistance and specific activity, but there is still a high need for improvement of transglutaminase for reasons such as high utility and further expansion of use. Therefore, it is an object of the present invention to address the issues of finding a novel mutation effective for the improvement of transglutaminase and providing a highly useful modified transglutaminase (variant), uses thereof, and the like.

### MEANS FOR SOLVING THE PROBLEM

In order to achieve the above-described object, the present inventors have attempted to improve a transglutaminase derived from Streptomyces mobaraensis by an amino acid substitution (by converting a specific amino acid residue to another amino acid). After trial and error, the present inventors have successfully identified a plurality of mutations (combinations of amino acid residues and amino acids after substitution) effective for a change in their properties (an improvement of high-temperature reactivity and a decrease in pH stability in the range of weak acidity).

By the way, in light of the common technical knowledge that enzymes of the same type have high similarity in structure (primary structure, steric structure) and it is highly probable that similar mutations produce similar effects, when a useful mutation found in a transglutaminase derived from Streptomyces mobaraensis having the amino acid sequence of SEQ ID NO: 1 is applied to another transglutaminase having high structural similarity with the above-described transglutaminase, there is a high probability that an identical effect is produced, and those skilled in the art can recognize that such application is effective.

The following invention is based on the above results and considerations.
[1] A modified transglutaminase having an amino acid sequence containing any one of following amino acid substitutions (1) to (11) in an amino acid sequence of SEQ ID NO: 1, or an amino acid sequence having 80% or more of identity to the amino acid sequence (provided that a difference in the amino acid sequence occurs at a position other than the position of the amino acid substitution), wherein a change in properties corresponding to the amino acid substitution(s) is observed:
   (1) amino acid substitutions at mutation points Y34 and F305, wherein an amino acid after the substitution at the mutation point Y34 is W, an amino acid after the substitution at the mutation point F305 is W, and a change in properties due to the amino acid substitutions is an improvement of high-temperature reactivity;
   (2) an amino acid substitution at a mutation point of M288, wherein an amino acid after the substitution is L, F, or Y, and a change in properties due to the amino acid substitution is a decrease in the pH stability in the range of weak acidity;
   (3) an amino acid substitution at a mutation point of D3, wherein an amino acid after the substitution is W or K, and a change in properties due to the amino acid substitution is an improvement of high-temperature reactivity;
   (4) amino acid substitutions at mutation points of D3 and F305, wherein an amino acid after the substitution at the mutation point D3 is G, K, N, P, or W, an amino acid after the substitution at the mutation point F305 is W, and a change in properties due to the amino acid substitutions is an improvement of high-temperature reactivity;
   (5) amino acid substitutions at mutation points V65 and F305, wherein an amino acid after the substitution at the mutation point V65 is I, an amino acid after the substitution at the mutation point F305 is W, and a change in properties due to the amino acid substitutions is an improvement of high-temperature reactivity;
   (6) amino acid substitutions at mutation points S303 and F305, wherein an amino acid after the substitution at the mutation point S303 is R or K, an amino acid after the substitution at the mutation point F305 is W, and a change in properties due to the amino acid substitutions is an improvement of high-temperature reactivity;
   (7) an amino acid substitution at a mutation point of R5, wherein an amino acid after the substitution is H, and a change in properties due to the amino acid substitution is a decrease in the pH stability in the range of weak acidity;
   (8) an amino acid substitution at a mutation point of V6, wherein an amino acid after the substitution is D, and a change in properties due to the amino acid substitution is a decrease in the pH stability in the range of weak acidity;
   (9) an amino acid substitution at a mutation point of W59, wherein an amino acid after the substitution is T, and a change in properties due to the amino acid substitution is a decrease in the pH stability in the range of weak acidity;
   (10) an amino acid substitution at a mutation point of S61, wherein an amino acid after the substitution is G or R, and a change in properties due to the amino acid substitution is a decrease in the pH stability in the range of weak acidity;
   (11) an amino acid substitution at a mutation point of V290, wherein an amino acid after the substitution is I, and a change in properties due to the amino acid substitution is a decrease in the pH stability in the range of weak acidity;
[2] The modified transglutaminase according to [1], wherein the identity is 82% or more.
[3] The modified transglutaminase according to [1], wherein the identity is 85% or more.
[4] The modified transglutaminase according to [1], wherein the identity is 90% or more.
[5] The modified transglutaminase according to [1], consisting of an amino acid sequence of any of SEQ ID NOs: 2 to 22.
[6] A gene encoding the modified transglutaminase according to any one of [1] to [5].
[7] The gene according to [6], including a nucleotide sequence of any of SEQ ID NOs: 23 to 43.
[8] A recombinant DNA including the gene according to [6] or [7].
[9] A microorganism having the recombinant DNA according to [8].
[10] An enzyme preparation containing the modified transglutaminase according to any one of [1] to [5].
[11] A method for preparing a modified transglutaminase, including the following steps (I) to (III):
   (I) a step of providing a nucleic acid encoding the amino acid sequence of the modified transglutaminase of any one of [1] to [5];
   (II) a step of expressing the nucleic acid; and
   (III) a step of recovering an expressed product.
[12] The preparing method according to [11], wherein the amino acid sequence is an amino acid sequence of any of SEQ ID NOs: 2 to 22.
[13] The preparing method according to [12], wherein the nucleic acid includes a nucleotide sequence of any of SEQ ID NOs: 23 to 43.

### EMBODIMENTS OF THE INVENTION

For convenience of description, some of the terms used in relation to the present invention are defined below.

### (Terms)

The term "modified transglutaminase" refers to an enzyme obtained by modifying or mutating a transglutaminase as a reference (hereinafter referred to as "reference transglutaminase"). The reference transglutaminase is typically a transglutaminase derived from Streptomyces mobaraensis having the amino acid sequence of SEQ ID NO: 1.

The term "transglutaminase derived from Streptomyces mobaraensis" refers to a transglutaminase whose origin is Streptomyces mobaraensis, and encompasses a transglutaminase produced by Streptomyces mobaraensis, and a transglutaminase expressed in another microorganism or the like with use of genetic information of the transglutaminase produced by Streptomyces mobaraensis.

In the present invention, "an amino acid substitution" is made as a modification or a mutation. Therefore, when a modified transglutaminase and a reference transglutaminase are compared, a difference is observed in some amino acid residues. In the present specification, a modified transglutaminase is also referred to as a modified enzyme or a variant.

In the specification, amino acids are designated according to common practice, as their single letters as described below, respectively:
methionine: M, serine: S, alanine: A, threonine: T, valine: V, tyrosine: Y, leucine: L, asparagine: N, isoleucine: I, glutamine: Q, proline: P, aspartic acid: D, phenylalanine: F, glutamic acid: E, tryptophan: W, lysine: K, cysteine: C, arginine: R, glycine: G, histidine: H

In the present specification, the position of a mutation point is identified by referring to the numbers assigned to amino acid residues of a maturated transglutaminase, in an ascending order from the amino acid residue at the N-terminus to the C-terminus of the maturated transglutaminase with the N-terminal amino acid residue as the 1-position.

According to common practice, an amino acid residue to be substituted, that is, a "mutation point", is represented by a combination of one character expressing the type of the amino acid and a numeral expressing the position of the amino acid. In addition, a mutation due to an amino acid substitution is represented by a combination of the indication of the mutation point and, on its right side, one character expressing the type of an amino acid after substitution. Therefore, for example, when the aspartic acid at the 3-position is a mutation point, it is expressed as "D3", and when the aspartic acid at the 3-position is a mutation point substituted with glycine, it is expressed as "D3G".

In the present specification, the term "range of weak acidity" refers to a range of pH 4 or more and pH 5 or less, and the term "neutral range" refers to a range of more than pH 5 and less than pH 8.

### 1. Modified transglutaminase

A first aspect of the present invention relates to a modified transglutaminase (hereinafter referred to as "modified enzyme"). The modified enzyme of the present invention typically has an amino acid sequence containing one or more specific amino acid substitutions (mutations) in an amino acid sequence of SEQ ID NO: 1. It is recognized that, due to this properties, a change in properties such as "an improvement of high-temperature reactivity" or "a decrease in pH stability in the range of weak acidity" occur in the transglutaminase consisting of the amino acid sequence of SEQ ID NO: 1. The modified enzyme having improved high-temperature reactivity exhibits high activity even at high temperature, and this is advantageous for application of the enzyme to, for example, foods and food raw materials produced under high-temperature conditions (for example, 60°C to 80°C). On the other hand, a modified enzyme having reduced pH stability in the range of weak acidity can be deactivated along with a decrease in pH of a food or the like to which the modified enzyme is applied, and thus has high utility value, for example, in applications for producing lactic acid fermented food products (yoghurt as a specific example). More specifically, when a modified enzyme having reduced pH stability in the range of weak acidity is used for the production of a lactic acid fermented food product, lactic acid fermentation may be performed after the modified enzyme is allowed to act on a fermentation raw material, or lactic acid fermentation may proceed simultaneously with the modified enzyme acting on the fermentation raw material. Incidentally, the amino acid sequence of SEQ ID NO: 1 is the sequence of a transglutaminase derived from Streptomyces mobaraensis.

In the present specification, the phrase "contain an amino acid substitution" means that an amino acid at a mutation point (that is, a position of an amino acid residue at which a specific amino acid substitution occurs) is an amino acid after substitution. Therefore, when the amino acid sequence mutated so as to include an amino acid substitution (mutated amino acid sequence) is compared with the amino acid sequence of SEQ ID NO: 1 (reference amino acid sequence) not including an amino acid substitution, any difference is recognized regarding the amino acid residue at the position of the amino acid substitution.

The high-temperature reactivity can be evaluated, for example, based on an activity calculated by an activity measurement method shown in Examples described below (note that the reaction temperature is changed from 37°C to 60°C). The modified enzyme having improved high-temperature reactivity has an activity at 60°C higher than that of the reference transglutaminase. The activity of the modified enzyme at 60°C is, for example, 180% or more, preferably 200% or more, more preferably 250% or more, and particularly preferably 270% or more of the activity of the reference transglutaminase.

The decrease in pH stability in the range of weak acidity can be evaluated, for example, based on the residual activity when a treatment at 30°C for 60 minutes at pH 4.0 to 5.0 is performed (the details of the measurement and evaluation of the residual activity are shown in Examples described below). In the modified enzyme having reduced pH stability in the range of weak acidity, the activity is remarkably lowered as the pH is lowered, in the range of weak acidity (specifically, in the range of pH 4.0 to pH 5.0). Therefore, it is recognized that the residual activity ratio is more abruptly lowered that that of the reference transglutaminase. The residual activity ratio of the modified enzyme having reduced pH stability in the range of weak acidity is 0% by the treatment at pH 4.0, and preferably 0% even by the treatment at pH 4.5.

The modified enzyme having reduced pH stability in the range of weak acidity is more useful than the reference transglutaminase in terms of the changed properties, but it is preferable that the activity is high in a neutral range (for example, pH 6) where the activity is expected to be exhibited, for example, in order to enable reduction in the amount used (enzyme amount). For example, the activity at pH 6 of the modified enzyme having reduced pH stability in the range of weak acidity is preferably 30% or more, more preferably 50% or more, still more preferably 80% or more, and even more preferably 100% or more (that is, the enzyme shows an activity equivalent to or higher than that of the reference transglutaminase) of the activity of the reference transglutaminase.

Hereinafter, the amino acid substitutions (mutation points and amino acids after the substitution) that cause each change in properties described above are listed.

### <Amino acid substitution effective for improvement of high-temperature reactivity>

(1) amino acid substitutions at mutation points Y34 and F305, wherein an amino acid after the substitution at the mutation point Y34 is W, and an amino acid after the substitution at the mutation point F305 is W;
(3) an amino acid substitution at a mutation point of D3, wherein an amino acid after the substitution is W or K;
(4) amino acid substitutions at mutation points D3 and F305, wherein an amino acid after the substitution at the mutation point D3 is G, K, N, P, or W, and an amino acid after the substitution at the mutation point F305 is W;
(5) amino acid substitutions at mutation points V65 and F305, wherein an amino acid after the substitution at the mutation point V65 is I, and an amino acid after the substitution at the mutation point F305 is W; and
(6) amino acid substitutions at mutation points S303 and F305, wherein an amino acid after the substitution at the mutation point S303 is R or K, and an amino acid after the substitution at the mutation point F305 is W.

Among the above amino acid substitutions, the following amino acid substitutions are more preferable amino acid substitutions because the degree of the improvement of high-temperature reactivity is large:
a double mutation of D3P and F305W;
a double mutation of D3W and F305W; and
a double mutation of Y34W and F305W.

### <Amino acid substitution effective for decreasing pH stability in range of weak acidity>

(2) an amino acid substitution at a mutation point of M288, wherein an amino acid after the substitution is L, F, or Y;
(7) an amino acid substitution at a mutation point of R5, wherein an amino acid after the substitution is H;
(8) an amino acid substitution at a mutation point of V6, wherein an amino acid after the substitution is D;
(9) an amino acid substitution at a mutation point of W59, wherein an amino acid after the substitution is T;
(10) an amino acid substitution at a mutation point of S61, wherein an amino acid after the substitution is G or R;
(11) an amino acid substitution at a mutation point of V290, wherein an amino acid after the substitution is I.

Among the above amino acid substitutions, the following amino acid substitutions are more preferable amino acid substitutions because the degree of the decrease in pH stability in the range of weak acidity is large:
M288L
M288Y

Incidentally, S61G, S61R, M288F, M288L, and V290I are highly useful in that they exhibit high activity in the neutral range (pH 6) in addition to the decrease in pH stability in the range of weak acidity.

Specific examples of the modified enzyme of the present invention may include a transglutaminase comprising an amino acid sequence of any of SEQ ID NOs: 2 to 22 (corresponding to D3G variant, D3K variant, D3W variant, D3G/F305W variant, D3K/F305W variant, D3N/F305W variant, D3P/F305W variant, D3W/F305W variant, Y34W/F305W variant, V65I/F305W variant, S303K/F305W variant, S303R/F305W variant, R5H variant, V6D variant, W59T variant, S61G variant, S61R variant, M288F variant, M288L variant, M288Y variant, V290I variant, respectively, in the stated order).

In general, when a part of the amino acid sequence of a certain protein is mutated, the mutated protein may have a function equivalent to that of the protein before the mutation. That is, the mutation of the amino acid sequence does not substantially affect the function of the protein, and the function of the protein before mutation may be maintained after the mutation. On the other hand, when the amino acid sequences of two proteins have high identity, it is highly probable that both proteins exhibit equivalent properties. In view of these common general technical knowledge, an enzyme can be regarded as being substantially identical to the above-described modified enzyme (a substantially identical transglutaminase) as long as the enzyme has an amino acid sequence that exhibits, not complete identity (i.e., 100% identity), but high identity to the amino acid sequence of the above-described modified enzyme, i.e. "the amino acid sequence containing any one of amino acid substitutions (1) to (11) in an amino acid sequence of SEQ ID NO: 1 described above (specific examples of the said amino acid sequence are the amino acid sequences of SEQ ID NOs: 2 to 22) " and is recognized to exhibit a desired change in properties. The identity herein is 70% or more, 80% or more, 82% or more, 85%, or more, 90% or more, 93% or more, 95% or more, 98%, or alternatively, 99% or more. A higher identity is more preferable. Therefore, in the most preferred aspect, the identity is 99% or more.

When the modified enzyme and the substantially identical transglutaminase are compared, a slight difference in amino acid sequence is to be recognized. It should be noted that the above-described difference in the amino acid sequence shall occur at a position other than the position where the above-described amino acid substitution is made. Therefore, the difference of the amino acid sequence occurs at, for example, the following position: a position other than the 3-position G in the case where the reference for the identity is the amino acid sequence of SEQ ID NO: 2; a position other than the 3-position K in the case where the reference for the identity is the amino acid sequence of SEQ ID NO: 3; a position other than the 3-position W in the case where the reference for the identity is the amino acid sequence of SEQ ID NO: 4; a position other than the 3-position G and the 305-position W in the case where the reference for the identity is the amino acid sequence of SEQ ID NO: 5; a position other than the 3-position K and the 305-position W in the case where the reference for the identity is the amino acid sequence of SEQ ID NO: 6; a position other than the 3-position N and the 305-position W in the case where the reference for the identity is the amino acid sequence of SEQ ID NO: 7; a position other than the 3-position P and the 305-position W in the case where the reference for the identity is the amino acid sequence of SEQ ID NO: 8; a position other than the 3-position W and the 305-position W in the case where the reference for the identity is the amino acid sequence of SEQ ID NO: 9; a position other than the 34-position W and the 305-position W in the case where the reference for the identity is the amino acid sequence of SEQ ID NO: 10; a position other than the 65-position I and the 305-position W in the case where the reference for the identity is the amino acid sequence of SEQ ID NO: 11; a position other than the 303-position K and the 305-position W in the case where the reference for the identity is the amino acid sequence of SEQ ID NO: 12; a position other than the 303-position R and the 305-position W in the case where the reference for the identity is the amino acid sequence of SEQ ID NO: 13; a position other than the 5-position H in the case where the reference for the identity is the amino acid sequence of SEQ ID NO: 14; a position other than the 6-position D in the case where the reference for the identity is the amino acid sequence of SEQ ID NO: 15; a position other than the 59-position T in the case where the reference for the identity is the amino acid sequence of SEQ ID NO: 16; a position other than the 61-position G in the case where the reference for the identity is the amino acid sequence of SEQ ID NO: 17; a position other than the 61-position R in the case where the reference for the identity is the amino acid sequence of SEQ ID NO: 18; a position other than the 288-position F in the case where the reference for the identity is the amino acid sequence of SEQ ID NO: 19; a position other than the 288-position L in the case where the reference for the identity is the amino acid sequence of SEQ ID NO: 20; a position other than the 288-position Y in the case where the reference for the identity is the amino acid sequence of SEQ ID NO: 21; and a position other than the 290-position I in the case where the reference for the identity is the amino acid sequence of SEQ ID NO: 22. In other words, in the amino acid sequence having the above identity (70% or more, 80% or more, 82% or more, 85% or more, 90% or more, 93% or more, 95% or more, 98%, or 99% or more) with the amino acid sequence of SEQ ID NO: 2, the amino acid at the 3-position is G. Similarly, in the amino acid sequence having the above identity with the amino acid sequence of SEQ ID NO: 3, the amino acid at the 3-position is K; in the amino acid sequence having the above identity with the amino acid sequence of SEQ ID NO: 4, the amino acid at the 3-position is W; in the amino acid sequence having the above identity with the amino acid sequence of SEQ ID NO: 5, the amino acid at the 3-position is G and the amino acid at the 305-position is W; in the amino acid sequence having the above identity with the amino acid sequence of SEQ ID NO: 6, the amino acid at the 3-position is K and the amino acid at the 305-position is W; in the amino acid sequence having the above identity with the amino acid sequence of SEQ ID NO: 7, the amino acid at the 3-position is N and the amino acid at the 305-position is W; in the amino acid sequence having the above identity with the amino acid sequence of SEQ ID NO: 8, the amino acid at the 3-position is P and the amino acid at the 305-position is W; in the amino acid sequence having the above identity with the amino acid sequence of SEQ ID NO: 9, the amino acid at the 3-position is W and the amino acid at the 305-position is W; in the amino acid sequence having the above identity with the amino acid sequence of SEQ ID NO: 10, the amino acid at the 34-position is W and the amino acid at the 305-position is W; in the amino acid sequence having the above identity with the amino acid sequence of SEQ ID NO: 11, the amino acid at the 65-position is I and the amino acid at the 305-position is W; in the amino acid sequence having the above identity with the amino acid sequence of SEQ ID NO: 12, the amino acid at the 303-position is K and the amino acid at the 305-position is W; in the amino acid sequence having the above identity with the amino acid sequence of SEQ ID NO: 13, the amino acid at the 303-position is R and the amino acid at the 305-position is W; in the amino acid sequence having the above identity with the amino acid sequence of SEQ ID NO: 14, the amino acid at the 5-position is H; in the amino acid sequence having the above identity with the amino acid sequence of SEQ ID NO: 15, the amino acid at the 6-position is D; in the amino acid sequence having the above identity with the amino acid sequence of SEQ ID NO: 16, the amino acid at the 59-position is T; in the amino acid sequence having the above identity with the amino acid sequence of SEQ ID NO: 17, the amino acid at the 61-position is G; in the amino acid sequence having the above identity with the amino acid sequence of SEQ ID NO: 18, the amino acid at the 61-position is R; in the amino acid sequence having the above identity with the amino acid sequence of SEQ ID NO: 19, the amino acid at the 288-position is F; in the amino acid sequence having the above identity with the amino acid sequence of SEQ ID NO: 20, the amino acid at the 288-position is L; in the amino acid sequence having the above identity with the amino acid sequence of SEQ ID NO: 21, the amino acid at the 288-position is Y; and in the amino acid sequence having the above identity with the amino acid sequence of SEQ ID NO: 22, the amino acid at the 290-position is I.

The "slight difference in amino acid sequence" herein is caused by amino acid deletion, substitution, addition, insertion, or a combination thereof. Typically, it means that a mutation (change) has occurred in the amino acid sequence due to deletion or substitution of one to several (the upper limit is, for example, 3, 5, 7, or 10) amino acids constituting the amino acid sequence, or addition or insertion of one to several (the upper limit is, for example, 3, 5, 7, or 10) amino acids, or a combination thereof. A "slight difference in amino acid sequence" preferably occurs due to conservative amino acid substitution. The term "conservative amino acid substitution" as used herein refers to substitution of a certain amino acid residue with an amino acid residue having a side chain having similar properties. Amino acid residues are classified into several families according to their side chains, such as basic side chains (e.g. lysine, arginine, histidine), acidic side chains (e.g., aspartic acid and glutamic acid), uncharged polar side chains (e.g. glycine, asparagine, glutamine, serine, threonine, tyrosine, cysteine), nonpolar side chains (e.g. alanine, valine, leucine, isoleucine, proline, phenylalanine, methionine, tryptophan), β-branched side chains (e.g. threonine, valine, isoleucine), and aromatic side chains (e.g. tyrosine, phenylalanine, tryptophan, histidine). The conservative amino acid substitution is substitution of an amino acid residue with another one in the same family. Since it is known that the active residue of a transglutaminase (SEQ ID NO: 1) derived from Streptomyces mobaraensis is cysteine at the 64-position, it is preferable that this acid residue is not affected when mutation is performed.

Incidentally, the identity (%) between two amino acid sequences or two nucleotide sequences (as a term encompasses these, "two sequences" is used hereinafter) can be determined by, for example, the following procedure. First, the two sequences are arranged so as to allow optimal comparison (for example, gaps may be introduced into the first sequence to optimize alignment with the second sequence). When a molecule (amino acid residue or nucleotide) at a specific position in the first sequence is the same as a molecule at a corresponding position in the second sequence, it can be said that the molecules at that position are the same. The identity of two sequences is a function of the number of identical positions common to the two sequences (that is, identity (%) = number of identical positions/total number of positions × 100), and preferably, the number and size of gaps required for optimization of alignment are also taken into consideration.

The comparison between two sequences and the determination of identity can be realized using a mathematical algorithm. Specific examples of the mathematical algorithm available for comparing sequences include the algorithms described in Karlin and Altschul (1993) Proc. Natl. Acad. Sci. USA 90: 5873-77. Such algorithms are incorporated into the NBLAST and XBLAST programs (version 2.0) described in Altschul et al. (1990) J. Mol. Biol. 215: 403-10. In order to obtain an equivalent nucleotide sequence, for example, BLAST nucleotide search may be performed using the NBLAST program with score = 100 and wordlength = 12. In order to obtain an equivalent amino acid sequence, for example, BLAST polypeptide search may be performed using the XBLAST program with score = 50 and wordlength = 3. To obtain gap alignments for comparison, Gapped BLAST is available as described in Altschul et al. (1997) Amino Acids Research 25 (17): 3389-3402. When utilizing BLAST and Gapped BLAST, the default parameters of the corresponding programs (e.g., XBLAST and NBLAST) can be used. For details, refer to http://www.ncbi.nlm.nih.gov. Examples of other mathematical algorithms available for comparing sequences include those described in Myers and Miller (1988) Comput Appl Biosci.4: 11-17. Such an algorithm is incorporated into, for example, the ALIGN program available in the GENESTREAM network server (IGH Montpellier, France) or the ISREC server. When the ALIGN program is used to compare amino acid sequences, for example, the PAM 120 residue mass table can be used, and gap length penalty = 12 and gap penalty = 4 can be set.

The identity of two amino acid sequences can be determined by using the GAP program of the EMBOSS package using the Blosum 62 matrix, with a gap weight = 10 and a gap length weight = 2. In addition, the identity of two nucleotide sequences can be determined by using the GAP program of the EMBOSS package (available in http://emboss.open-bio.org/), with a gap weight = 50 and a gap length weight = 3.

Typically, a transglutaminase consisting of the amino acid sequence of SEQ ID NO: 1, that is, a transglutaminase derived from Streptomyces mobaraensis, is mutated (any of the amino acid substitution(s) according to (1) to (11) described above) to provide a modified enzyme of the present invention. The substantially identical transglutaminase described above can be obtained by any of the following: adding a further mutation to a product obtained by mutation (amino acid substitution(s) according to any of (1) to (11) above) of a transglutaminase consisting of the amino acid sequence of SEQ ID NO: 1; adding an equivalent mutation to a transglutaminase consisting of an amino acid sequence having high identity with the amino acid sequence of SEQ ID NO: 1, such as a transglutaminase derived from a genus homologous to a Streptomyces mobaraensis strain that produces a transglutaminase consisting of the amino acid sequence of SEQ ID NO: 1; or adding a further mutation to those obtained by the mutation. In the "equivalent mutation" herein, in an amino acid sequence having high identity with the amino acid sequence of SEQ ID NO: 1, an amino acid residue equivalent to an amino acid residue at a mutation point in the present invention (a mutation point relating to any of the amino acid substitution(s) according to (1) to (11) described above is to be substituted). Incidentally, examples of the transglutaminase consisting of the amino acid sequence having high identity with the amino acid sequence of SEQ ID NO: 1 include: a transglutaminase that is derived from Streptomyces mobaraensis (formerly classified as Streptoverticillium ladakanum) and has an amino acid sequence of SEQ ID NO: 44 (amino acid sequence identity: 93%); a transglutaminase that is derived from Streptomyces albireticuli and has the amino acid sequence of SEQ ID NO: 45 (amino acid sequence identity: 82%); a transglutaminase that is derived from Streptomyces luteireticuli and has the amino acid sequence of SEQ ID NO: 46 (amino acid sequence identity: 82%); a transglutaminase that is derived from Streptomyces cinnamoneus and has the amino acid sequence of SEQ ID NO: 47 (amino acid sequence identity: 81%); a transglutaminase that is derived from Streptomyces platensis and has the amino acid sequence of SEQ ID NO: 48 (amino acid sequence identity: 80%); and a transglutaminase that is derived from Streptomyces hygroscopicus and has the amino acid sequence of SEQ ID NO: 49 (amino acid sequence identity: 80%).

The term "equivalent", when used in conjunction with an amino acid residue in the present specification, means that, for proteins (enzymes) compared, the amino acid residues thereof make identical contribution to the exercise of the function thereof. For example, when an amino acid sequence to be compared is arranged with respect to an amino acid sequence of a reference transglutaminase (amino acid sequence of SEQ ID NO: 1) so that optimal comparison is allowed while partial homology of a primary structure (amino acid sequence) is taken into consideration (at this time, gaps may be introduced as necessary to optimize alignment), an amino acid at a position corresponding to a specific amino acid in the reference amino acid sequence can be specified as an "equivalent amino acid". It is also possible to specify the "equivalent amino acid" by comparing steric structures (three-dimensional structures), instead of or in addition to comparing primary structures. By using the steric structure information, a highly reliable comparison result can be obtained. In this case, a method of performing alignment while comparing atomic coordinates of steric structures of a plurality of enzymes can be adopted. The steric structure information of the enzyme to be mutated can be obtained, for example, from the Protein Data Bank (http://www.pdbj .org/index#j .html).

An example of a method for determining a protein steric structure by X-ray crystallography is shown below.
(1) A protein is crystallized. Crystallization is essential to determine a steric structure, and in addition, crystallization is industrially useful as a purification method of a protein at high purity and a stable preservation method of a protein at high density. In this case, a protein to which a substrate or an analog compound thereof is bound as a ligand may be crystallized.
(2) The prepared crystal is irradiated with X ray to collect diffraction data. There are many cases that a protein crystal is damaged due to X ray irradiation and the diffraction ability is deteriorated. In such cases, a low-temperature measurement technique of rapidly cooling the crystal to about -173°C and collecting diffraction data in the state has been recently prevailed. In addition, ultimately, synchrotron orbit radiation having high luminance is utilized to collect high resolution data that is used for structure determination.
(3) In addition to the diffraction data, phase information is necessary in order to perform the crystal structure analysis. When a crystal structure of an analogous protein to a desired protein is unknown, it is impossible to determine the structure in a molecular substitution method, and a phase problem has to be solved by a heavy-atom isomorphous replacement method. The heavy-atom isomorphous replacement method is a method in which a metallic atom having a high atomic number such as mercury or platinum is introduced into a crystal and contribution of a large X ray scattering ability of such a metallic atom to X ray diffraction data is utilized to collect phase information. The determined phase is possibly improved by smoothing an electron density of a solvent region in the crystal. Since a water molecule in the solvent region has large fluctuation, the electron density is hardly observed, and thus adjusting the electron density in this region to close to 0 makes it possible to approach the real electron density, which results in improving a phase. In addition, when plural molecules are contained in an asymmetrical unit, equation of electron densities of these molecules makes it possible to more significantly improve a phase. A model of a protein is fit to an electron density map calculated using the phase improved as described above. This process is performed on computer graphics using a program such as QUANTA made by MSI Co. (USA). After the process, structural precision is performed using a program such as X-PLOR made by MSI to complete the structure analysis. When a crystal structure of an analogous protein to a desired protein is known, it can be determined in a molecular substitution method using the atomic coordinate of the known protein. Molecular substitution and structural precision can be performed using a program such as CNS#SOLVE ver.11.

### 2. Nucleic acid encoding modified transglutaminase, etc.

A second aspect of the present invention provides a nucleic acid relating to a modified enzyme of the present invention. That is, provided are a gene that encodes a modified enzyme, a nucleic acid that can be used as a probe for identifying a nucleic acid that encodes the modified enzyme, and a nucleic acid that can be used as a primer for causing amplification, mutating, etc. to a nucleic acid that encodes the modified enzyme.

The gene that encodes a modified enzyme is typically used in preparation of the modified enzyme. A genetic engineering preparation method using the gene that encodes a modified enzyme makes it possible to obtain a modified enzyme in a more uniform state. Further, the method can be a suitable method also in the case of preparing a large amount of a modified enzyme. Note that uses of the gene that encodes a modified enzyme are not limited to the preparation of the modified enzyme. For example, the nucleic acid can also be used as a tool for an experiment intended for clarification of action mechanisms of a modified enzyme or a tool for designing or preparing a further modified one of the enzyme.

The "gene that encodes a modified enzyme" herein refers to such a nucleic acid that, when it is expressed, the modified enzyme can be obtained, and encompasses a nucleic acid having a nucleotide sequence corresponding to the amino acid sequence of the modified enzyme, and in addition, a nucleic acid obtained by adding, to such a nucleic acid, a sequence that does not encode an amino acid sequence. Degeneracy of a codon is also considered.

Examples of a sequence (nucleotide sequence) of a gene that encodes a modified enzyme include those of SEQ ID NOs: 23 to 43 shown below. These sequences encode variants shown in the Examples below.
SEQ ID NO: 23: D3G variant;
SEQ ID NO: 24: D3K variant;
SEQ ID NO: 25: D3W variant;
SEQ ID NO: 26: D3G/F305W variant;
SEQ ID NO: 27: D3K/F305W variant;
SEQ ID NO: 28: D3N/F305W variant;
SEQ ID NO: 29: D3P/F305W variant;
SEQ ID NO: 30: D3W/F305W variant;
SEQ ID NO: 31: Y34W/F305W variant;
SEQ ID NO: 32: V65I/F305W variant;
SEQ ID NO: 33: S303K/F305W variant;
SEQ ID NO: 34: S303R/F305W variant;
SEQ ID NO: 35: R5H variant;
SEQ ID NO: 36: V6D variant;
SEQ ID NO: 37: W59T variant;
SEQ ID NO: 38: S61G variant;
SEQ ID NO: 39: S61R variant
SEQ ID NO: 40: M288F variant;
SEQ ID NO: 41: M288L variant;
SEQ ID NO: 42: M288Y variant; and
SEQ ID NO: 43: V290I variant.

When the gene of the present invention is expressed in a host, usually, in order to stabilize the structure of a modified enzyme which is an expressed product, a gene construct in which a sequence encoding a propeptide (pro-sequence) is added to the 5' terminus side of the above sequence (for example, any of SEQ ID NOs: 23 to 43) is introduced into the host. When the gene is expressed as a secretory protein, a gene contract is further prepared in which a sequence encoding a pre-sequence (signal sequence) is added to the 5' terminus side of a sequence encoding a pro-sequence. When the foregoing gene construct is used, after it is expressed as a prepro-type transglutaminase to which a pre-sequence and a pro-sequence are linked, cleavage of the pre-sequence (conversion to a pro-type transglutaminase) and cleavage of the pro-sequence occur, whereby a maturated transglutaminase is obtained. As the sequence encoding the pre-sequence and the sequence encoding the pro-sequence, the original sequence, that is, the sequence of the reference transglutaminase (transglutaminase before modification) may be used. A specific example of the pre-sequence is a sequence of SEQ ID NO: 51 (a pre-sequence of a transglutaminase derived from Streptomyces mobaraensis). A specific example of the pro-sequence is a sequence of SEQ ID NO: 52 (a pro-sequence of a transglutaminase derived from Streptomyces mobaraensis). A specific example of a sequence encoding the pre-sequence is a sequence of SEQ ID NO: 53 (a sequence encoding a pre-sequence of a transglutaminase derived from Streptomyces mobaraensis). A specific example of a sequence encoding a pro-sequence is a sequence of SEQ ID NO: 54 (a sequence encoding a pro-sequence of a transglutaminase derived from Streptomyces mobaraensis).

The nucleic acid of the present invention can be prepared in an isolated state by use of a standard genetic engineering technique, a molecular biological technique, a biochemical technique, a chemical synthesis, and the like in reference to the present specification or the sequence information disclosed in the appended sequence listing.

In another aspect of the present invention, a nucleic acid is provided that is different in a nucleotide sequence in a part when compared to the nucleotide sequence of the gene that encodes the modified enzyme of the present invention, although functions of proteins encoded by these are identical (such a nucleic acid is hereinafter also referred to as a "homologous nucleic acid", and a nucleotide sequence defining a homologous nucleic acid is also referred to as a "homologous nucleotide sequence"). An example of the homologous nucleic acid includes a DNA that is composed of a nucleotide sequence including substitution, deletion, insertion, addition, or inversion of one to several bases on the basis of the base sequence of the nucleic acid encoding the modified enzyme of the present invention and that encodes a protein having an enzyme activity typical of the modified enzyme (i.e., transglutaminase activity). Substitution or deletion of bases may occur in a plurality of sites. The "plurality" herein means, for example, 2 to 40 bases, preferably 2 to 20 bases, and more preferably 2 to 10 bases, though depending on positions or kinds of amino acid residues in a steric structure of a protein encoded by the nucleic acid.

The homologous nucleic acid has an identity of, for example, 60% or more, preferably 70% or more, more preferably 80% or more, still more preferably 85% or more, further more preferably about 90% or more, still further preferably 95% or more, and most preferably 99% or more, to the nucleotide sequence as a reference.

Such a homologous nucleic acid as described above can be obtained by, for example, a restriction enzyme treatment, a treatment with exonuclease, DNA ligase, etc., or introduction of mutation by a site directed mutation introduction method (Molecular Cloning, Third Edition, Chapter 13, Cold Spring Harbor Laboratory Press, New York), and a random mutation introduction method (Molecular Cloning, Third Edition, Chapter 13, Cold Spring Harbor Laboratory Press, New York). A homologous nucleic acid can be obtained also in other methods such as exposure to ultraviolet radiation.

Another aspect of the present invention relates to a nucleic acid having a nucleotide sequence complementary to the nucleotide sequence of the gene that encodes the modified enzyme of the present invention. Still another aspect of the present invention provides a nucleic acid having a nucleotide sequence with an identity of at least about 60%, 70%, 80%, 90%, 95%, 99%, or 99.9% to the nucleotide sequence of the gene that encodes the modified enzyme of the present invention or the nucleotide sequence complementary to the foregoing nucleotide sequence.

Still another aspect of the present invention relates to a nucleic acid having a nucleotide sequence that hybridizes to a nucleotide sequence complementary to the nucleotide sequence of the gene that encodes the modified enzyme of the present invention or its homologous nucleotide sequence under stringent conditions. The "stringent conditions" herein refer to conditions wherein a so-called specific hybrid is formed and a nonspecific hybrid is not formed. Such stringent conditions are known to a person skilled in the art and can be set in reference to, for example, Molecular Cloning (Third Edition, Cold Spring Harbor Laboratory Press, New York) and Current protocols in molecular biology (edited by Frederick M. Ausubel et al., 1987). Examples of the stringent conditions include conditions of using a hybridization liquid (50% formamide, 10×SSC (0.15 M NaCl, 15 mM sodium citrate, pH 7.0), a 5× Denhardt solution, 1% SDS, 10% dextran sulfate, 10 µg/ml of modified salmon sperm DNA, and a 50 mM phosphate buffer (pH7.5)) and incubating at about 42°C to about 50°C, thereafter washing with 0.1×SSC and 0.1% SDS at about 65°C to about 70°C.

Still another aspect of the present invention provides a nucleic acid (nucleic acid fragment) having a part of the nucleotide sequence of the gene that encodes the modified enzyme of the present invention or the nucleotide sequence complementary to the foregoing nucleotide sequence. Such a nucleic acid fragment can be used in detection, identification, and/or amplification of a nucleic acid having the nucleotide sequence of the gene that encodes the modified enzyme of the present invention. For example, the nucleic acid fragment is designed so as to at least include a part being hybridized to a sequential nucleotide moiety (for example, about 10 to about 100 bases length, preferably about 20 to about 100 bases length, more preferably about 30 to about 100 bases length) in the nucleotide sequence of the gene that encodes the modified enzyme of the present invention. When used as a probe, the nucleic acid fragment can be labeled. A fluorescent substance, an enzyme, a radioactive isotope or the like can be used for the labeling.

Still another aspect of the present invention relates to a recombinant DNA including the gene of the present invention (the gene that encodes a modified enzyme). The recombinant DNA of the present invention is provided in, for example, a form of a vector. The term "vector" in the present specification refers to such a nucleic acid molecule that can transfer a nucleic acid inserted in the vector to a target such as a cell.

An appropriate vector is selected according to its intended use (cloning, expression of a protein) and in consideration of a kind of a host cell. Examples include a M13 phage or a modified form thereof, a λ phage or a modified form thereof, and pBR322 or a modified form thereof (e.g., pB325, pAT153, pUC8), pET21, etc. as a vector having Escherichia coli as a host; pYepSecl, pMFa, pYES2, pPIC3.5K, etc. as a vector having a yeast as a host; pAc, pVL, etc. as a vector having an insect cell as a host; and pCDM8, pMT2PC, etc. as a vector having a mammal cell as a host.

The vector of the present invention is preferably an expression vector. The "expression vector" refers to a vector capable of introducing a nucleic acid inserted in the expression vector into a target cell (host cell) and causing the nucleic acid to be expressed in the cell. The expression vector generally includes a promoter sequence necessary for expression of a nucleic acid inserted, an enhancer sequence for promoting expression, and the like. An expression vector including a selection marker can also be used. When such an expression vector is used, the presence or absence (and its degree) of introduction of the expression vector can be confirmed using a selection marker.

Insertion of the nucleic acid of the present invention into the vector, insertion of a selection marker gene (if necessary), insertion of a promoter (if necessary), and the like can be performed by using a standard recombinant DNA technique (for example, a known method of using a restriction enzyme and a DNA ligase, which can be referred in Molecular Cloning, Third Edition, 1.84, Cold Spring Harbor Laboratory Press, New York).

From the viewpoint of ease of handling, the host cell can be microorganisms such as an aspergillus (e.g., Aspergillus oryzae), a bacillus bacterium (e.g., Bacillus subtilis, Bacillus licheniformis, or Bacillus amyloliquefaciens), a brevibacillus bacterium (e.g., Brevibacillus choshinensis), E. coli (Escherichia coli) and budding yeast (Saccharomyces cerevisiae). Any host cell can be used so long as it allows replication of the recombinant DNA and expression of the gene of the modified enzyme. Preferably, E. coli (Escherichia coli) and budding yeast (Saccharomyces cerevisiae) can be used. A microorganism of the genus Streptomyces (e.g., Streptomyces mobaraensis) can also be used as the host. An example of E. coli can be E. coli BL21 (DE3) when a T7 promoter is used, and can be E. coli JM109 otherwise. Examples of budding yeast can include budding yeast SHY2, budding yeast AH22 and budding yeast INVScl (Invitrogen).

Another aspect of the present invention relates to a microorganism having the recombinant DNA of the present invention (that is, a transformant). The microorganism of the present invention can be obtained by transfection or transformation using the vector of the invention described above. The transfection or transformation can be performed by, for example, the calcium chloride method (J. Mol. Biol., 53, 159 (1970)), the Hanahan method (J. Mol. Biol., 166, 557 (1983)), the SEM method (Gene, 96, 23 (1990)), a method by Chung, et al. (Proc. Natl. Acad. Sci. U.S.A. 86, 2172 (1989)), the calcium phosphate coprecipitation method, electroporation (Potter, H. et al., Proc. Natl. Acad. Sci. U.S.A. 81, 7161-7165 (1984)), and lipofection (Felgner, P. L. et al., Proc. Natl. Acad. Sci. U.S.A. 84, 7413-7417 (1984)). The microorganism of the present invention can be used for producing the modified enzyme of the present invention.

### 3. Enzyme preparation containing modified transglutaminase

The modified enzyme of the present invention is provided, for example, in the form of an enzyme preparation. The enzyme preparation may contain, in addition to the active ingredient (modified enzyme of the present invention), an excipient, a buffer, a suspending agent, a stabilizer, a preservative, an antiseptic, physiological saline, various proteins, degradation products of various proteins, various extracts, various salts, various antioxidants, cysteine, glutathione, sodium glutamate, sodium inosinate, sodium guanylate, calcined shell calcium, silicon dioxide, or the like. As the excipient, starch, dextrin, maltose, trehalose, lactose, D-glucose, sorbitol, D-mannitol, sucrose, glycerol and the like can be used. As the buffer agent, phosphates, citrates, acetates, and the like can be used. As the stabilizer, propylene glycol, ascorbic acid, and the like can be used. As the preservative, phenol, benzalkonium chloride, benzyl alcohol, chlorobutanol, methylparaben, and the like can be used. As the antiseptic, ethanol, benzalkonium chloride, paraoxybenzoic acid, chlorobutanol, and the like can be used. Examples of proteins include soy protein, wheat protein, corn protein, milk protein, and animal-derived protein. Examples of extracts include meat extracts, plant extracts, and yeast extracts. Examples of salts include chloride salts, phosphates, polyphosphates, pyrophosphates, citrates, lactates, and carbonates. Examples of antioxidants include L-ascorbic acid salts, and sodium bisulfite. The shape of the enzyme preparation of the present invention is not particularly limited, and is, for example, powder, granule, liquid, capsule or the like.

### 4. Methods for preparation of modified transglutaminase

A further aspect of the present invention relates to a method for preparation of a modified enzyme. In an aspect of the method for preparation according to the present invention, the modified enzyme of the present invention is prepared by a genetic engineering procedure. In this aspect, a nucleic acid encoding the amino acid sequence of the modified enzyme of the present invention (for example, any of SEQ ID NOs: 2 to 22) is prepared (step (I)). Here, a "nucleic acid encoding a particular amino acid sequence" gives, upon its expression, a polypeptide having the amino acid sequence encoded thereby, and includes not only a nucleic acid consisting of a nucleotide sequence corresponding to the amino acid sequence, but also a nucleic acid that may have an additional sequence added thereto, which may or may not encode an amino acid sequence. Degeneracy of a codon is also considered. The "nucleic acid encoding the amino acid sequence of the modified enzyme of the present invention" can be prepared in an isolated state by use of a standard genetic engineering technique, a molecular biological technique, a biochemical technique, and the like in reference to the present specification or the sequence information disclosed in the appended sequence listing. Here, the amino acid sequence of the modified enzyme of the present invention results from mutation of the amino acid sequence of the reference transglutaminase. Therefore, "a nucleic acid encoding the amino acid sequence of the modified enzyme of the present invention" can also be obtained by applying a required mutation to the gene encoding the reference transglutaminase. A large number of methods for site-specific nucleotide sequence substitution are known in the art (see, for example, Molecular Cloning, Third Edition, Cold Spring Harbor Laboratory Press, New York), and from among these, suitable methods can be selected and used. As a site-specific mutagenesis method, site-specific saturation mutagenesis of amino acids can be employed. The site-specific saturation mutagenesis of amino acids is a "Semi-rational, semi-random" method in which the position involved in the desired function of a given protein is estimated on the basis of the steric structure of the protein, which is then subjected to amino acid saturation mutagenesis (J. Mol. Biol. 331, 585-592 (2003)). For example, site-specific saturation mutagenesis of amino acids can be performed by using kits, such as Quick change (Stratagene), and overlap extension PCR (Nucleic Acid Res. 16, 7351-7367 (1988)). As the DNA polymerase for use in the PCR, for example, Taq polymerase can be employed. Preferably, high-accuracy DNA polymerases such as KOD-PLUS- (Toyobo Co., Ltd.) and Pfu turbo (Stratagene) are used.

Following the step (I), the prepared nucleic acid is expressed (step (II)). For example, firstly, an expression vector inserted with the above described nucleic acid is prepared and a host cell is transformed using this constructed vector.

Then, a transformant is cultured under the condition of producing a modified enzyme that is an expressed product. Culture of the transformant may follow a general method. An assimilable carbon compound may be used as a carbon source used for a medium. For example, glucose, sucrose, lactose, maltose, molasses, or pyruvic acid is used. An available nitrogen compound may be used as a nitrogen source. For example, peptone, meat extract, yeast extract, casein hydrolysate, or soybean bran alkali extract is used. Other than those substances, phosphate, carbonate, sulfate, salts of magnesium, calcium, potassium, iron, manganese and zinc, specific amino acids, specific vitamins, and the like are used according to necessity.

On the other hand, a culture temperature can be set within a range of 30°C to 40°C (preferably at around 33 to 37°C). A culture time can be set by considering growing properties of a transformant to be cultured and production properties of a modified enzyme. A pH of a medium is set within a range wherein a transformant grows and an enzyme is produced. The pH of a medium is preferably set at about 6.0 to 9.0 (preferably at around pH 7.0).

Subsequently, the expressed product (modified enzyme) is collected (step (III)). A culture liquid containing bacterial cells after culture may be used as an enzyme solution directly or after undergoing condensation, removal of impurities, or the like, but the expressed product is generally once collected from the culture liquid or bacterial cells. When the expressed product is a secretory protein, it can be collected from the culture liquid, and in other cases, the expressed product can be collected from bacterial cells. In the case of collecting from the culture liquid, for example, an undissolved substance is removed by filtration and centrifugation on a culture supernatant, and then, a purified product of a modified enzyme can be obtained by separation and purification in combination of vacuum concentration, membrane concentration, salting out using ammonium sulfate or sodium sulfate, fractional precipitation by methanol, ethanol, or acetone, dialysis, heating treatment, isoelectric treatment, various kinds of chromatography such as gel filtration, adsorption chromatography, ion exchange chromatography, and affinity chromatography (for example, gel filtration with Sephadex gel (GE Healthcare Life Sciences), etc., DEAE sepharose CL-6B (GE Healthcare Life Sciences), octyl sepharose CL-6B (GE Healthcare Life Sciences), CM sepharose CL-6B (GE Healthcare Life Sciences)). On the other hand, in the case of collecting the expressed product from bacterial cells, a culture liquid is subjected to filtration, centrifugation, or the like, to thus obtain the bacterial cells, then the bacterial cells are crushed by a mechanical method such as a pressure treatment and an ultrasonic treatment, or an enzymatic method with a lysozyme or the like, thereafter carrying out separation and purification in the same manner as described above, and a purified product of a modified enzyme can be thus obtained.

The purified enzyme obtained as described above can be provided after being powdered, for example, by freeze dry, vacuum dry, or spray dry. In this time, the purified enzyme may be previously dissolved in a phosphoric acid buffer solution, a triethanol amine buffer solution, a tris-hydrochloric acid buffer solution, or a GOOD buffer solution. Preferably, a phosphoric acid buffer solution and a triethanol amine buffer solution can be used. Note that, for the GOOD buffer solution herein, PIPES, MES or MOPS is exemplified.

Generally, genetic expression and collection of the expressed product (modified enzyme) are carried our using an appropriate host-vector system as described above, but a cell-free synthesis system may also be employed. Herein, the "cell-free synthesis system (cell-free transcription system, cell-free transcription/translation system)" refers to in vitro synthesis of mRNA or a protein from a nucleic acid (DNA or mRNA) being a template, which codes for the mRNA or the protein, using a ribosome, a transcription/translation factor derived from living cells (alternately, obtained in a genetic engineering technique) or the like, not using living cells. In the cell-free synthesis system, a cell extraction obtained from a cell disruptor that is purified according to necessity is generally used. The cell extraction generally includes ribosome necessary for protein synthesis and various factors such as an initiation factor, and various enzymes such as tRNA. When a protein is synthesized, this cell extraction is added with other substances necessary for protein synthesis, such as various amino acids, energy sources such as ATP and GTP, and creatine phosphate. As a matter of course, ribosome and various factors and/or various enzymes, and the like, which are separately prepared, may be supplemented if necessary in the protein synthesis.

Development of a transcription/translation system reconstructing various molecules (factors) necessary for protein synthesis has also been reported (Shimizu, Y. et al.: Nature Biotech., 19, 751-755, 2001). In this synthesis system, a gene of 31 kinds of factors composed of 3 kinds of initiation factors constituting a protein synthesis system of bacteria, 3 kinds of elongation factors, 4 kinds of factors associated with termination, 20 kinds of aminoacyl tRNA synthesis enzymes that make each amino acid combine with tRNA, and a methionyl tRNA formyl transfer enzyme is amplified from an Escherichia coli genome, and a protein synthesis system is reconstructed in vitro using them. Such a reconstructed synthesis system may be used in the present invention.

The term "cell-free transcription/translation system" is interchangeably used with a cell-free protein synthesis system, an in vitro translation system or an in vitro transcription/translation system. In the in vitro translation system, RNA is used as a template to synthesize a protein. Any of RNA, mRNA, an in vitro transcribed product, or the like is used as the template RNA. On the other hand, in the in vitro transcription/translation system, DNA is used as a template. The template DNA should include in a ribosome bonding region, and preferably contains a suitable terminator sequence. In addition, in the in vitro transcription/translation system, a condition of adding factors necessary for each reaction is established so that a transcription reaction and a translation reaction proceed sequentially.

### EXAMPLES

### <Search for effective mutation points to improve properties>

For obtaining highly useful modified transglutaminases, an attempt was made to improve the enzyme function (improvement of high-temperature reactivity, a decrease in pH stability in the range of weak acidity) of a transglutaminase derived from Streptomyces mobaraensis (wild type enzyme; SEQ ID NO: 1) by using protein engineering. First, for introducing a mutation due to an amino acid substitution, a CASTing library (for example, see Angew Chem Int Ed Engl. 2006 Feb 13; 45(8): 1236-41) and alanine scanning (Ala scanning) (for example, J Mol Biol 1995 Feb 17; 246(2): 317-30) were utilized to select candidate mutation points. Next, a mutation was introduced into each mutation point by the following method to prepare a mutated enzyme.

### 1. Preparation of mutated enzyme

### 1-1. Introduction of mutation

(1) A PCR primer was designed for introduction of a mutation.
(2) With use of a primer set for each mutation point, PCR was performed by using, as a template, plasmid pET20b in which transglutaminase gene (SEQ ID NO: 50) was incorporated (15 cycles of reactions at 98°C for 1 minute, at 98°C for 10 seconds, at 60°C for 15 seconds and at 68°C for 2 minutes were performed, and the resultant product was reacted at 68°C for 5 minutes, and then allowed to stand at 4°C).
(3) Dpnl (1.5 µL/tube) was added to the PCR reaction solution (25 µL/tube) for treatment (37°C, 3 hours or longer).
(4) Ligation treatment (16°C, overnight) was performed using T4 kinase.
(5) E. coli BL21 (DE3) was transformed with the ligation reaction solution (11 µL/tube), and cultured in an LB medium containing ampicillin (37°C, overnight).

### 1-2. Obtainment of enzyme extract

(1) A strain into which a mutation was introduced (mutated strain) is inoculated into a TB medium containing ampicillin, and culture at 33°C for 48 hours. IPTG (final concentration: 0.1 mM) was added 24 hours after the start of the culture.
(2) After centrifugation of the culture solution (3,000 g × 10 minutes), the supernatant was removed, and the bacterial cells are recovered.
(3) A bacteriolytic agent is added to lyse the bacterial cells.
(4) After centrifugation of the bacterial lysate (3,000 g x 10 minutes), the supernatant was recovered and used as an enzyme extract.

### 1-3. Maturation (removal of propeptide sequence)

(1) Equal amounts of the enzyme extract and a 2 mg/mL protease (Dispase) solution were mixed to cause a reaction (30°C, 2 hours or longer).
(2) After centrifugation of the mixed solution (3,000 g × 10 minutes), the supernatant was recovered and used as a maturated enzyme.

### 2. Purification of enzyme

Each maturated enzyme was purified by using TALON Spin columns (Takara Bio Inc.) and HisTALON Buffer Set (Takara Bio Inc.), according to the attached protocol. The purified maturated enzymes were used for evaluating properties of the mutated enzymes.

### 3. Evaluation of property of mutated enzyme

The following activity measurement methods were used to evaluate the properties of each prepared mutated enzyme.

### <Activity measurement method>

The maturated enzyme is diluted to an appropriate concentration with 200 mM Tris-HCl pH 6.0 (sample solution). To 10 µL of the sample solution, 100 µL of the substrate solution (R-1) was added, and the solution mixture is mixed and caused to react at 37°C for 10 minutes. A coloring solution (R-2) (100 µL) was added to stop the reaction and form an Fe complex, and then the absorbance at 525 nm was measured. As a control, the absorbance of a previously heat-inactivated enzyme solution subjected to a similar reaction was measured, and the absorbance difference thereof from the sample solution was determined. Separately, a calibration curve was prepared using L-glutamic acid-γ-monohydroxamic acid instead of the enzyme liquid, and the amount of hydroxamic acid generated from the absorbance difference was determined. The enzyme activity that produced 1 µmol of hydroxamic acid per minute was defined as one unit (1 U).

### (Substrate solution (R-1))

2.42 g of 2-Amino-2-hydroxymethyl-1.3-propanediol, 0.70 g of hydroxyammonium hydrochloride, 0.31 g of reduced glutathione, and 1.01 g of Z-Gln-Gly (benzyloxycarbonyl-L-glutaminylglycine) were dissolved in distilled water to attain a total volume of 100 mL (pH 6.0).

### (Substrate solution (R-2))

Mixed were 30 mL of 3M hydrochloric acid solution, 30 mL of 12% trichloroacetic acid solution, and 30 mL of 5% iron (III) chloride solution.

### 3-1. Evaluation of high-temperature reactivity

The maturated enzyme was diluted 5 times with 200 mM Tris-HCl pH 6.0 (sample solution), and the activity was measured at a reaction temperature of 60°C.

The activity at the reaction temperature of 60°C was compared with that of a wild type, and amino acid substitutions effective for improving the high-temperature reactivity were identified.

Fig. 1 shows the evaluation results regarding the high-temperature reactivity. All of the amino acid substitutions, resulting in an improvement of the high-temperature reactivity, were determined as effective amino acid substitutions. Particularly in the cases of D3P/F305W substitution, D3W/F305W substitution, and Y34W/F305W substitution, the activity was improved to 250% or more of that of the wild type, and these substitutions were determined to be particularly effective amino acid substitutions.

**[Table 1]**

| Mutation | | Activity at 60°C (U/g) |
|---|---|---|
| No mutation (wild type) | | 11 |
| D3G | F305W | 24 |
| D3K | - | 25 |
| D3K | F305W | 21 |
| D3N | F305W | 26 |
| D3P | F305W | 34 |
| D3W | - | 26 |
| D3W | F305W | 31 |
| Y34W | F305W | 30 |
| V65I | F305W | 20 |
| S303K | F305W | 24 |
| S303R | F305W | 21 |

### 3-2. Evaluation of pH stability

The maturated enzyme was diluted 2 times with 200 mM Britton-Robinson Buffer at each pH (pH 4, 4.5, 5, 5.5, 6), and treated at 30 °C for 60 minutes, to obtain pH-treated samples. The maturated enzyme was diluted 2 times with 200 mM Tris-HCl pH 6.0 to obtain untreated samples. The untreated samples and the pH-treated samples were diluted 2 times with 500 mM Tris-HCl pH 6.0, then activities were measured, and evaluation was made on relative activities thereof with respect to the activity of the untreated sample of the wild-type enzyme, which was assumed to be 100.

Amino acid substitutions resulting in relative activities of 0 at pH 4.0 or pH 4.5 were identified.

Table 2 shows the evaluation results regarding a decrease in pH stability in the range of weak acidity. All of the amino acid substitutions, resulting in relative activities at pH 4.0 of 0, were determined to be amino acid substitutions effective for decreasing pH stability in the range of weak acidity. In the case of the M288L substitution and the M288Y substitution, the relative activity at pH 4.5 was also 0, and thus it was determined to be a particularly effective amino acid substitution. The M288L substitution is highly useful also in that it shows high activity in the neutral range (pH 6). From the viewpoint of activity in the neutral range (pH 6), it can be said that the S61G substitution, the S61R substitution, the M288F substitution, and the V290I substitution are also highly useful.

**[Table 2]**

| Mutation | Relative activity to activity of wild-type enzyme w/o pH treatment assumed to be 100 | | | | | |
|---|---|---|---|---|---|---|
| | Untreated | pH4 | pH4.5 | pH5 | pH5.5 | pH6 |
| No mutation (wild type) | 100 | 73 | 103 | 109 | 107 | 120 |
| R5H | 42 | 0 | 35 | 43 | 39 | 67 |
| V6D | 37 | 0 | 38 | 28 | 36 | 52 |
| W59T | 60 | 0 | 47 | 64 | 85 | 82 |
| S61G | 114 | 0 | 53 | 98 | 131 | 120 |
| S61R | 166 | 0 | 127 | 182 | 121 | 145 |
| M288F | 122 | 0 | 36 | 95 | 133 | 139 |
| M288L | 116 | 0 | 0 | 58 | 103 | 126 |
| M288Y | 83 | 0 | 0 | 51 | 75 | 70 |
| V290I | 97 | 0 | 84 | 125 | 116 | 119 |

### INDUSTRIAL APPLICABILITY

The modified transglutaminase of the present invention has improved practically important properties, and has high industrial value. Therefore, the modified transglutaminase of the present invention is expected to be used in new applications as well as in existing applications.

The present invention is not limited to the description of the embodiments and examples of the invention. Various modifications that can be easily conceived by those skilled in the art without departing from the scope of the claims are also included in the present invention. The entire contents of the articles, published patent publications, patent publications, and the like specified in the present specification are incorporated herein by reference.

### SEQUENCE LISTING FREE TEXT

SEQ ID NO: 2: Explanation of artificial sequence: D3G variant
SEQ ID NO: 3: Explanation of artificial sequence: D3K variant
SEQ ID NO: 4: Explanation of artificial sequence: D3W variant
SEQ ID NO: 5: Explanation of artificial sequence: D3G/F305W variant
SEQ ID NO: 6: Explanation of artificial sequence: D3K/F305W variant
SEQ ID NO: 7: Explanation of artificial sequence: D3N/F305W variant
SEQ ID NO: 8: Explanation of artificial sequence: D3P/F305W variant
SEQ ID NO: 9: Explanation of artificial sequence: D3W/F305W variant
SEQ ID NO: 10: Explanation of artificial sequence: Y34W/F305W variant
SEQ ID NO: 11: Explanation of artificial sequence: V65I/F305W variant
SEQ ID NO: 12: Explanation of artificial sequence: S303K/F305W variant
SEQ ID NO: 13: Explanation of artificial sequence: S303R/F305W variant
SEQ ID NO: 14: Explanation of artificial sequence: R5H variant
SEQ ID NO: 15: Explanation of artificial sequence: V6D variant
SEQ ID NO: 16: Explanation of artificial sequence: W59T variant
SEQ ID NO: 17: Explanation of artificial sequence: S61G variant
SEQ ID NO: 18: Explanation of artificial sequence: S61R variant
SEQ ID NO: 19: Explanation of artificial sequence: M288F variant
SEQ ID NO: 20: Explanation of artificial sequence: M288L variant
SEQ ID NO: 21: Explanation of artificial sequence: M288Y variant
SEQ ID NO: 22: Explanation of artificial sequence: V290I variant
SEQ ID NO: 23: Explanation of artificial sequence: D3G variant
SEQ ID NO: 24: Explanation of artificial sequence: D3K variant
SEQ ID NO: 25: Explanation of artificial sequence: D3W variant
SEQ ID NO: 26: Explanation of artificial sequence: D3G/F305W variant
SEQ ID NO: 27: Explanation of artificial sequence: D3K/F305W variant
SEQ ID NO: 28: Explanation of artificial sequence: D3N/F305W variant
SEQ ID NO: 29: Explanation of artificial sequence: D3P/F305W variant
SEQ ID NO: 30: Explanation of artificial sequence: D3W/F305W variant
SEQ ID NO: 31: Explanation of artificial sequence: Y34W/F305W variant
SEQ ID NO: 32: Explanation of artificial sequence: V65I/F305W variant
SEQ ID NO: 33: Explanation of artificial sequence: S303K/F305W variant
SEQ ID NO: 34: Explanation of artificial sequence: S303R/F305W variant
SEQ ID NO: 35: Explanation of artificial sequence: R5H variant
SEQ ID NO: 36: Explanation of artificial sequence: V6D variant
SEQ ID NO: 37: Explanation of artificial sequence: W59T variant
SEQ ID NO: 38: Explanation of artificial sequence: S61G variant
SEQ ID NO: 39: Explanation of artificial sequence: S61R variant
SEQ ID NO: 40: Explanation of artificial sequence: M288F variant
SEQ ID NO: 41: Explanation of artificial sequence: M288L variant
SEQ ID NO: 42: Explanation of artificial sequence: M288Y variant
SEQ ID NO: 43: Explanation of artificial sequence: V290I variant

## Claims

1. A modified transglutaminase having an amino acid sequence containing any one of following amino acid substitutions (1) to (11) in an amino acid sequence of SEQ ID NO: 1, or an amino acid sequence having 80% or more of identity to the amino acid sequence (provided that a difference in the amino acid sequence occurs at a position other than the position of the amino acid substitution), wherein a change in properties corresponding to the amino acid substitution(s) is observed:
(1) amino acid substitutions at mutation points Y34 and F305, wherein an amino acid after the substitution at the mutation point Y34 is W, an amino acid after the substitution at the mutation point F305 is W, and a change in properties due to the amino acid substitutions is an improvement of high-temperature reactivity;
(2) an amino acid substitution at a mutation point of M288, wherein an amino acid after the substitution is L, F, or Y, and a change in properties due to the amino acid substitution is a decrease in the pH stability in the range of weak acidity;
(3) an amino acid substitution at a mutation point of D3, wherein an amino acid after the substitution is W or K, and a change in properties due to the amino acid substitution is an improvement of high-temperature reactivity;
(4) amino acid substitutions at mutation points of D3 and F305, wherein an amino acid after the substitution at the mutation point D3 is G, K, N, P, or W, an amino acid after the substitution at the mutation point F305 is W, and a change in properties due to the amino acid substitutions is an improvement of high-temperature reactivity;
(5) amino acid substitutions at mutation points V65 and F305, wherein an amino acid after the substitution at the mutation point V65 is I, an amino acid after the substitution at the mutation point F305 is W, and a change in properties due to the amino acid substitutions is an improvement of high-temperature reactivity;
(6) amino acid substitutions at mutation points S303 and F305, wherein an amino acid after the substitution at the mutation point S303 is R or K, an amino acid after the substitution at the mutation point F305 is W, and a change in properties due to the amino acid substitutions is an improvement of high-temperature reactivity;
(7) an amino acid substitution at a mutation point of R5, wherein an amino acid after the substitution is H, and a change in properties due to the amino acid substitution is a decrease in the pH stability in the range of weak acidity;
(8) an amino acid substitution at a mutation point of V6, wherein an amino acid after the substitution is D, and a change in properties due to the amino acid substitution is a decrease in the pH stability in the range of weak acidity;
(9) an amino acid substitution at a mutation point of W59, wherein an amino acid after the substitution is T, and a change in properties due to the amino acid substitution is a decrease in the pH stability in the range of weak acidity;
(10) an amino acid substitution at a mutation point of S61, wherein an amino acid after the substitution is G or R, and a change in properties due to the amino acid substitution is a decrease in the pH stability in the range of weak acidity;
(11) an amino acid substitution at a mutation point of V290, wherein an amino acid after the substitution is I, and a change in properties due to the amino acid substitution is a decrease in the pH stability in the range of weak acidity;

2. The modified transglutaminase according to claim 1, wherein the identity is 82% or more.

3. The modified transglutaminase according to claim 1, wherein the identity is 85% or more.

4. The modified transglutaminase according to claim 1, wherein the identity is 90% or more.

5. The modified transglutaminase according to claim 1, consisting of an amino acid sequence of any of SEQ ID NOs: 2 to 22.

6. A gene encoding the modified transglutaminase according to any one of claims 1 to 5.

7. The gene according to claim 6, including a nucleotide sequence of any of SEQ ID NOs: 23 to 43.

8. A recombinant DNA including the gene according to claim 6 or 7.

9. A microorganism having the recombinant DNA according to claim 8.

10. An enzyme preparation containing the modified transglutaminase according to any one of claims 1 to 5.

11. A method for preparing a modified transglutaminase, including the following steps (I) to (III):
(I) a step of providing a nucleic acid encoding the amino acid sequence of the modified transglutaminase of any one of claims 1 to 5;
(II) a step of expressing the nucleic acid; and
(III) a step of recovering an expressed product.

12. The preparing method according to claim 11, wherein the amino acid sequence is an amino acid sequence of any of SEQ ID NOs: 2 to 22.

13. The preparing method according to claim 12, wherein the nucleic acid includes a nucleotide sequence of any of SEQ ID NOs: 23 to 43.
